Europäisches Patentamt

European Patent Office

Office européen des brevets

(19)

(11) Publication number : **0 542 466 A2**

(12)

# EUROPEAN PATENT APPLICATION

(21) Application number : 92310085.3

(22) Date of filing : 04.11.92

(51) Int. Cl.⁵ : **C12N 15/86,** C12N 7/01, C12N 5/10, A61K 39/00, G01N 33/53

(30) Priority : **11.11.91 GB 9123929**

(43) Date of publication of application : **19.05.93 Bulletin 93/20**

(84) Designated Contracting States : **AT BE CH DE DK ES FR GB GR IE IT LI LU MC NL PT SE**

(71) Applicant : **THE WELLCOME FOUNDATION LIMITED Unicorn House 160 Euston Road London NW1 2BP (GB)**

(72) Inventor : **Peakman, Timothy Charles Langley Court Beckenham, Kent BR3 3BS (GB)** Inventor : **Gewert, Dirk Rolf Langley Court Beckenham, Kent BR3 3BS (GB)**

(74) Representative : **Stott, Michael John et al The Wellcome Foundation Limited Group Patents & Agreements Langley Court Beckenham Kent BR3 3BS (GB)**

(54) **Baculovirus transfer vectors.**

(57) A novel insect cell expression system.

EP 0 542 466 A2

Fig.1

This invention relates to a novel insect cell expression system.

Since the discovery and characterisation of baculoviruses, considerable use has been made of insect cells for the expression of heterologous genes. The advantages of this system lie in the high levels of expression which can be achieved, the ability of cells to modify recombinant proteins correctly and to assemble subunits into functional proteins (Luckow et al, Bio/Technology, 1988, 6, 47-55). Heterologous DNA sequences are usually cloned into transfer vectors under the transcriptional control of very late viral promoters, such as the promoter for the polyhedrin gene or the P10 gene. These genes are naturally expressed at very high levels and are non-essential for viral propagation in continuous culture. After insertion of the heterologous sequence into the transfer vector, it is then transferred into the virus by a process of homologous recombination between sequences flanking the polyhedrin gene in the virus and the modified polyhedrin gene in the transfer vector. The resulting recombinant has a deleted polyhedrin gene and is unable to synthesise polyhedrin occlusion bodies. This phenotype can be visually detected on a standard agarose overlay assay (Brown et al., J.Gen.Virol., 1977, 36, 361-364).

Significant progress has been made in the refinement of baculovirus vectors in order to optimise expression levels of foreign proteins. In particular, the sequences required for full activity of the polyhedrin promoter have been determined (Matsuura et al., J.Gen.Virol, 1987, 68, 1233-1250; and Ooi et al., J.Mol.Biol., 1989, 210, 721-736). Examples of vectors currently used for high level expression of non-fused proteins are p36C (Page, Nucleic Acids Res., 1989, 17, 454), pVL941 (Luckow et al., Virology, 1989, 170, 31-39) and pAcYMI (Matsuura et al., ibid). However, a major problem with the baculovirus/insect cell expression system is in the identification and isolation of recombinant viruses. This problem has been alleviated somewhat by the generation of dual expression vectors. In most cases, one transcription unit directs expression of the b-galactosidase gene and recombinants are identified on a standard agarose overlay assay by the blue plaque phenotype in the presence of the chromogenic indicator X-gal. An example of such a vector, pJVNheI, has been described previously (Vialard et al., J.Virol., 1990, 64, 37-50). However, the use of such vectors has led to the identification of a large number of "false" recombinants exhibiting the blue phenotype whilst remaining polyhedrin positive on an overlay assay. These viral progeny may have arisen by the integration of the transfer vector by a single homologous cross-over event resulting in tandemly repeated viral sequences. These viral progeny appear to be unstable in long term propagation. In addition, although dual expression vectors aid the identification of recombinant viruses, they do not improve the frequency of recombination between virus and transfer vector. Indeed, because the addition of a second transcription unit increases the size of the transfer vector, the frequency of transplacement may conceivably be reduced.

Another limitation to the wider application of the baculovirus/insect cell system for the expression of foreign proteins is the low efficiency of the homologous recombination, which is typically from $1:10^2$ to $1:10^4$ (depending on the size of the heterologous gene in the transfer vector, resulting in small total numbers of recombinant progeny virus. A significant step in improving the frequency of recombinants among viral progeny has been the observation that the use of linear viral DNA in the co-transfection procedure considerably reduces the background of non-recombinants (Kitts et al., Nucleic Acids Res., 1990, 18, 5668-5672). However, although the actual percentage of recombinants increases, the total number of viral progeny dramatically decreases. Furthermore, it relies on two double stranded homologous exchange events so that the efficiency of recombination could be expected to decrease as the size of the foreign gene increases.

A novel expression system has now been developed which is based on baculovirus infection of insect cells to obtain expression of heterologous genes. In particular, the novel expression system utilises the Cre (for "Causes REcombination) recombinase enzyme and its substrate loxP (for "Locus of Crossover PI"). The expression system thus provides an efficient in vitro system for construction of recombinant baculoviruses that can be identified and isolated. Indeed, the system enables frequencies of recombinant baculoviruses to be obtained that may be as high as $5x10^7$ recombinants per microgram of starting plasmid DNA, up to 50% of the viral progeny being recombinants. Moreover, heterologous genes inserted into the baculovirus genome may be readily recovered in a simple one-step process and re-inserted after manipulation if required. Furthermore, this recombination is independent of the size of the heterologous gene.

Accordingly, the present invention provides a baculovirus transfer vector, which contains a restriction site for insertion of a DNA sequence encoding an heterologous polypeptide, regulatory DNA elements to provide for expression of the DNA sequence, when inserted into the restriction site, and a loxP DNA sequence to act as a substrate for the Cre recombinase protein.

The Cre-loxP system has been characterised (Sternberg et al., J.Mol.Biol., 1981, 467-486). Bacteriophage PI encodes a site-specific recombination system that consists of a site (lox) at which recombination occurs and a gene, Cre, the protein product of which is essential for the recombination. The Cre protein has been purified and cloned and the sequence and structure of lox determined (Abremski et al., J.Biol.Chem., 1984, 1509-1514; and Hoess et al., P.N.A.S., 1984, 81, 1026-1029). The interaction between Cre and its substrate lox, has been studied using nuclease protection techniques. The region protected against DNAseI attack is a 34bp sequence containing two 13bp perfect inverted repeats separated by an 8bp non-palindromic spacer (Hoess, ibid). These sequences have been shown to be sufficient for Cre mediated recombination. No high energy co-factors are required and the Cre protein binds to lox containing DNA to form a complex. The bound Cre converts about 70% of DNA substrate to products in a stoichiometric manner. Significantly, the action of Cre on a super-coiled substrate containing two lox sites reversably generates product molecules that are topologically unlinked.

The loxP DNA sequence of use with the present invention is as follows, or is substantially the same as that which follows:

5' CCTTAATATAACTTCGTATAATGTATGCTATACGAAGTTATTAGGTCG 3'

GGAATTATATTGAAGCATATTACATACGATATGCTTCAATAATCCAGC

The sequence may be obtained by synthesis or cloning (Hoess et al., Proc.Nat.Acad.Sci., 1984, 81, 1026-1029). The sequence may be contained within the transfer vector in a position upstream or downstream of the restriction site, or even in a different orientation relative to the restriction site, as long as it does not substantially interfere with the DNA regulatory elements.

The Cre recombinase protein of use with the present invention preferably has an apparent molecular weight on SDS of 35kDa and an amino acid analysis:

| Amino acid | Mol residue/mol Cre | Amino acid | Mol residue/mol Cre |
|---|---|---|---|
| Ala | 32 | Lys | 12 |
| Arg | 30 | Met | 8 |
| Asx | 36 | Phe | 8 |
| Glx | 28 | Pro | 9 |
| Gly | 21 | Ser | 20 |
| His | 6 | Thr | 15 |
| Ile | 13 | Tyr | 6 |
| Leu | 31 | Val | 19 |

in which tryptophan and cysteine were not determined and the calculations were based on the assumption that the protein contains 294 residues (Abremski et al., J.Biol.Chem., 1984, 259(3), 1509-1514).

The protein may be isolated from cultures of E.coli infected with bacteriophage P1 or may be prepared using recombinant DNA technology. As a further alternative, it may be obtained from DuPont (UK) Ltd., Biotechnology Systems Divisions, Wedgewood Way, Stevenage, Herts. SG1 4QN.

The restriction site is preferably unique to the vector, and may constitute any known site.

The DNA sequence encoding an heterologous polypeptide of use with the present invention may be of any size. For example, sequences up to about 10 kB or even higher may be used with the present invention. The heterologous polypeptide itself may be an antigenic polypeptide of use in diagnostic assays or in vaccines or may be a therapeutic polypeptide of use in human or animal medicine.

The regulatory DNA elements of use with the present invention preferably comprise a promoter and other sequences required for transcription and/or translation of the heterologous gene. Preferred examples of a promoter include the polyhedrin promoter and the P10 promoter.

The recombinant baculoviruses obtained using the transfer vector of the present invention may be screened by Southern blots using the heterologous gene. It is however preferred that the transfer vector also contains a DNA sequence encoding a selectable phenotype. The use of such a sequence enables the recombinant baculoviruses to be readily identified and isolated. Prefered examples of a DNA sequence encoding a selectable phenotype include tbe b-galactosidase gene which allows a visual selection of viral progeny after transfection.

The baculovirus transfer vector of the present invention may be constructed by appropriately engineering known or available transfer vectors using standard techniques in the art.

3

The present invention also provides a recombinant DNA molecule, comprising a baculovirus transfer vector of the present invention and a DNA sequence encoding an heterologous polypeptide inserted into the restriction site of the vector.

The DNA sequence encoding an heterologous polypeptide may be inserted into the restriction site of the vector using standard techniques known in the art.

The present invention also provides a baculovirus which contains a loxP DNA sequence to act as a substrate for the Cre recombinase protein.

Examples of a baculovirus for use with the present invention include Autographa californica nuclear polyhedrosis virus (AcNPV).

The loxP DNA sequence may be inserted into the genome of the baculovirus using a transfer vector, such as p36C (Page, Nucleic Acids Res., 1989, 17, 454), followed by transfection into insect cells.

The present invention also provides a recombinant baculovirus produced by site-specific recombination of baculovirus, which contains a loxP DNA sequence to act as a substrate for the Cre recombinase protein, and a recombinant DNA molecule of the present invention in the presence of the Cre recombinase protein.

Such site-specific recombination may be carried out in a buffer at elevated temperature using, for example, from 1 to 10 units of the Cre recombinase protein.

The present invention also provides an insect cell transfected with a recombinant baculovirus of the present invention.

Examples of an insect cell for use with the present invention include Spodoptera frugiperda.

Transfection may be carried out using Transfectam (Stratagene Ltd., Cambridge Innovation Centre, Cambridge Science Park, Milton Road, Cambridge, CB4 4GF).

The present invention also provides a process for the production of an heterologous polypeptide, which comprises culturing an insect cell transfected in accordance with the present invention.

Culture of the transfected insect cell may be carried out using standard techniques known in the art. The heterologous polypeptide may subsequently be isolated.

The novel expression system of the present invention enables large numbers of recombinant baculoviruses to be obtained. These can then be readily screened using a selectable marker as described herein. The expression system also provides a means for high-level expression of cDNAs and especially a means of expression screening. Desired recombinants may thus be both screened and produced using this system.

Once isolated, the polypeptide may be pumped according to standard procedures of the art, including ammonium sulphate precipitation, affinity columns, column chromatography, gel electrophoresis and the like (See, generally, Scopes, R., Protein Purification, Springer-Verlag, N.Y., (1982)

Once purified the polypeptide may then be used therapeutically in or in developing and performing assay procedures or immunoassays, immunofluorescent staining, and the like (See, generally Immunological Methods, Vols I and II, Lefkovits and Pernis, eds., Academic Press, New York, N.Y., (1979 and 19781)), or in a vaccine.

The heterologous polypeptide produced by the aforementioned process may be incorporated into a vaccine for conferring immunity. For this purpose the heterologous polypeptide produced according to the process of the invention may be presented in association with a physiologically acceptable carrier or diluent possibly in admixture with other agents. Typically carriers and diluents are sterile, pyrogen-free liquid media suitable as vehicles for introducing a polypeptide into a patient. Suitable carriers include but are not limited to physiological saline, phosphate buffered saline, phosphate buffered saline glucose and buffered saline.

The vaccine may also comprise an adjuvant for stimulating the immune response and thereby enhance the effect of the vaccine. A convenient adjuvant is aluminium hydroxide.

Also included within the invention are formulations containing a polypeptide produced according to the aforementioned process. Conveniently, the vaccines are formulated to contain a final concentration of polypeptide of from 0.2 to 200lg/ml, preferably 5 to 50lg/ml, most preferably about 30lg/ml. After formulation, the vaccine may be incorporated into a sterile container which is then sealed and stored at a low temperature, for example 4°C, or it may be freeze-dried.

The vaccines may be administered by the conventional method for the administration of vaccines including oral and parenteral (e.g. subcutaneous or intramuscular) injection. The treatment may consist of a single dose of vaccine or a plurality of doses over a period of time. It is recommended that each dose is 0.1 to 2ml preferably 0.2 to 1ml, most preferably about 0.5ml of vaccine.

The present invention further relates to a method of determining the present of antibodies to a polypeptide made according to the aforementioned process in a human body fluid comprising:

(i) contacting a solid phase to which is immobilised the polypeptide which is expressed in insect cells with a test sample;

(ii) determining whether the said polypeptide has bound to any said antibodies.

An immunoassay for carrying out such a detection method may comprise polypeptide produced by the process described herein for contacting with the bodily sample and means for detecting the polypeptide specific antibodies that bind to the polypeptide

The present invention also relates to a method for determining the presence of a polypeptide antigen made according to the process described herein in a human body fiuid comprising.

(i) contacting a solid phase to which are immobilised antibodies reactive with polypeptide expressed in insect cells with a test sample;

(ii) determining whether the said antibodies have bound to any said polypeptide antigen.

An immunoassay for carrying out such a detection method may comprise polypeptide antibodies raised against the polypeptide produced by the process described herein and means for detecting the polypeptide antigens that bind to the antibodies.

A test sample of any appropriate physiological fluid may be used in the assay, for example urine, plasma, blood, serum, semen, tears, saliva or cerebrospinal fluid.

A variety of assay formats may be employed. The antigen can be used to capture selectively antibody against polypeptide from solution, to label selectively such antibody already captured, or to both capture and label. In addition the antigen may be used in a variety of homogeneous assay formats in which the antibodies which react with the antigen are detected in solution with no separation of phases. The antigen can also be used for polypeptide antigen detection.

The type of assay in which the antigen is used to capture antibodies from solution involve immobilisation of the antigen onto a solid surface. This surface should be capable of being washed in some way. The sort of surfaces which may be used are polymers of various types (moulder into microtitre wells; beads; dipsticks of various types; aspiration tips; electrodes; and optical devices), particles (for example latex; stabilised blood, bacterial or fungal cells; spores; gold or other metallic sols; and proteinaceous colloids; with the usual size of the particle being from 0.1 to 5 microns), membranes (for example nitrocellulose; paper; cellulose acetate; and high porosity/high surface area membranes of an organic or inorganic material).

The attachment of the antigen to the surface can be by passive adsorption from a solution of optimum composition which may include surfactants, solvents, salts, chaotropes; or by active chemical bonding. Active bonding may be through a variety of reactive or activatible functional groups which may be attached to the surface (for example condensing agents; active esters, halides, anhydrides; amino, hydroxyl, or carboxyl groups; sulphydryl groups; carbonyl groups; diazo groups; unsaturated groups).

Alternatively the active bonding may be through a polypeptide (itself attached to the surface passively or through active bonding) or through a carrier protein such as albumin or casein, to which the antigen may be chemically bonded by any of a variety of methods and which may confer advantages because of isoelectric point, charge, hydrophilicity or other physico-chemical property. The antigen may also be attached to the surface (usually but not necessarily a membrane) following electrophoretic separation of a reaction mixture e.g. an immune precipitation.

After contacting (reacting) the surface bearing the antigen with a test sample and removing the excess of the sample where necessary by any of a variety of means (washing, centrifugation, filtration, magnetism, capilliary action), the captured antibody is detected by a revealing label, any means which will give a detectable signal. For example, this may be achieved by use of a labelled molecule or particle as defined above which will react with the captured antibody (for example protein A or protein G and the like; anti-species or anti-immunoglobulin-sub-type; rheumatoid factor; antibody to the antigen used in a competitive or blocking fashion; or any molecule containing an epitope of the antigen including the antigen itself and other proteins and peptides derived directly or indirectly from HCMV)

The detectable signal may be optical or radio-active or physico-chemical, provided by directly labelling the molecule referred to with for example a dye, radiolabel, electroactive species, magnetically resonant species or fluorophore; or indirectly by labelling the molecule or particle with an enzyme itself capable of giving rise to a measurable change of any sort. Alternatively the detectable signal may be due to, for example, agglutination, diffraction effect or birefringent effect occurring if any of the surfaces referred to are particles.

Those types of assay in which an antigen is used to label an already captured antibody require some form of labelling of the antigen which will allow it to be detected.

The labelling can be direct, by chemically or passively attaching for example a radio-, magnetic resonant-, particle or enzyme label to the antigen; or indirect by attaching any form of label to a molecule which will itself react with the antigen e.g. antibody to the antigen; with subsequent reaction of the labelled molecule with the antigen.

The chemistry of bonding a label can be directly through a moiety already present in the antigen such as an amino group or through an inserted group such as a maleimide. Capture of the antibody may be on any of the surfaces already mentioned, by any reagent, including passive or activated adsorption, which will result in specific antibody or immune complexes being bound. In particular capture of the antibody could be by anti-species or anti-immunoglobulin-sub-type, by rheumatoid factor, proteins A, G and the like, or by any molecule containing the epitope making up the antigen as described above.

For those assays in which the antigen is used to provide a measure of the polypeptide antigen in a sample, the antigen may be labelled in any of the ways described above, and used in either a competitive binding fashion so that its binding by any specific molecule on any of the surfaces exemplified above is blocked by antigen in the sample, or in a non-competitive fashion when antigen in the sample is bound specifically or non-specifically to any of the surfaces above, in turn binds a specific bi- or poly-valent molecule (e.g. an antibody) and the remaining valencies of the molecule are used to capture the labelled antigen.

In general in homogeneous assays the antigen and an antibody are labelled, so that, when the antibody reacts with the antigen in free solution, the two labels interact, for example to allow non-radiative transfer of energy captured by one 'label to the other label, with appropriate detection of the excited second label or quenched first label (e.g. by fluorimetry, magnetic resonance or enzyme measurement). Addition of either antigen or antibody in a sample results in restriction of the interaction of the labelled pair, and so to a different level of signal in the detector.

A suitable assay format for detecting polypeptide antibody is the direct sandwich enzyme immunoassay (EIA) format. The polypeptide is coated onto microtitre wells. A test sample and polypeptide to which an enzyme is coupled (conjugated protein) are added simultaneously. Any specific antibody binds both to the polypeptide coating the well and to the conjugated antigen. Typically the same antigen is used on both sides of the sandwich. After washing, bound enzyme is detected using a specific substrate involving a colour change. A test kit for use in such an EIA comprises:

(1) polypeptide produced by the process contained herein, labelled with an enzyme;
(2) a substrate for the enzyme;
(3) means providing a surface on which polypeptide is immobilised; and
(4) optionally, washing solutions and/or buffers.

In the accompanying drawings:

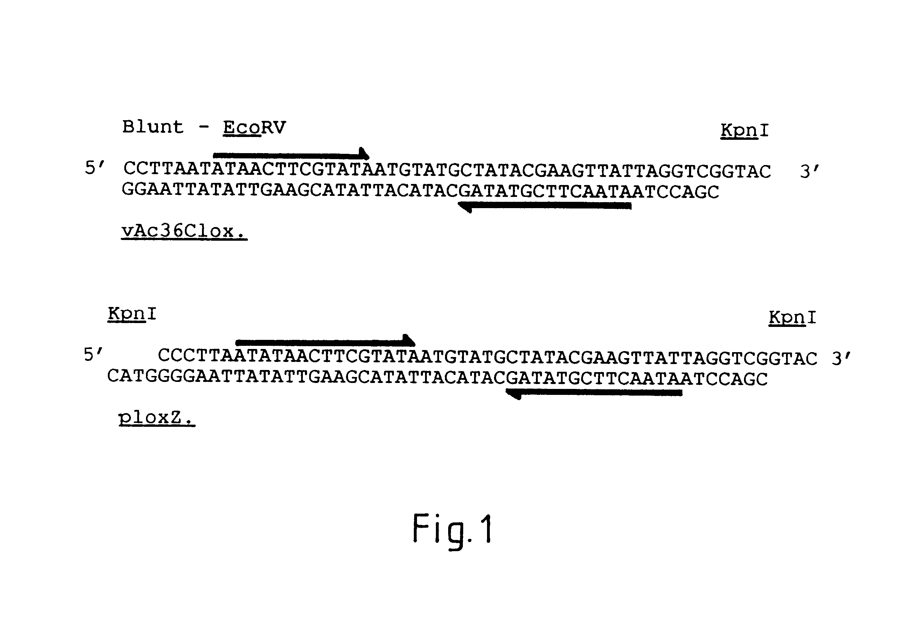

Figure 1 provides the sequences of the oligonucleotide pairs used to construct p36Clox and vAclox (vAc-36Clox) and pKSlox (ploxZ). The lox sites, consisting of two 13bp perfect inverted repeats separated by an 8bp spacer are indicated by a half arrow.

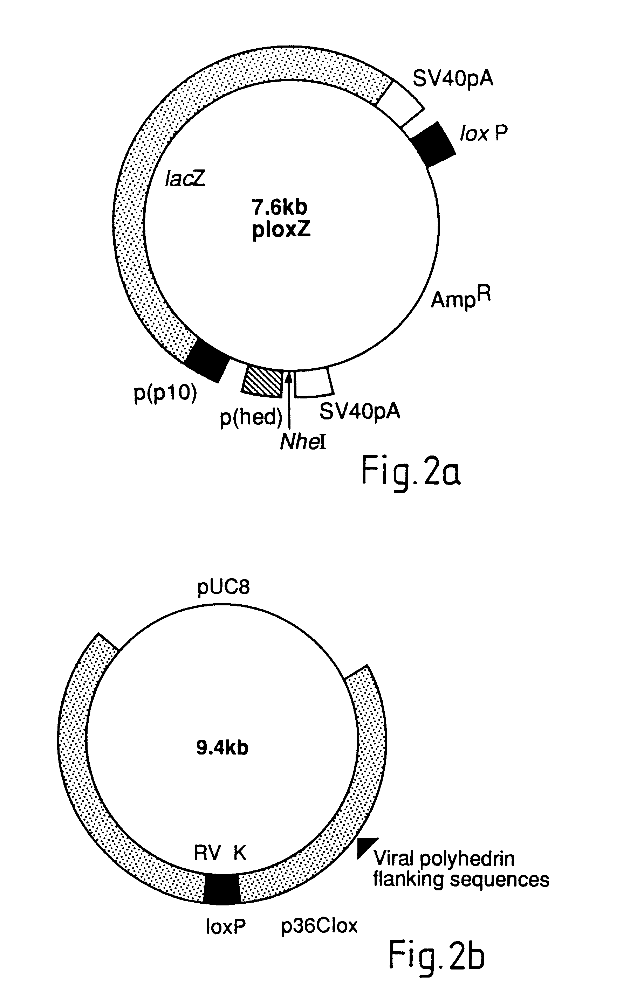

Figure 2a provides the structure of plasmid ploxZ. The plasmid backbone is Bluescript+, a standard cloning vector available from Stratagene Ltd., Cambridge Innovation Centre, Cambridge Science Park, Milton Road, Cambridge CB4 4GF. Expression of the b-galactosidase coding sequence (lacZ) is controlled by the very late viral P10 promoter [p(P10)] and transcribed in a clockwise direction. Genes of interest are cloned into the unique NheI site and are transcribed in the counter-clockwise direction by the very late viral polyhedrin promoter [p(hed)]. Polyadenylation signals from SV40 (SV40pA) direct processing of both genes. The lox site (loxP) is cloned downstream of the b-galactosidase expression cassette.

Figure 2b provides the structure of p36Clox. The polyhedrin sequences between the EcoRV (RV) site and the KpnI (K) site of p36C have been deleted and replaced with a loxP site using the oligonucleotides shown in Figure 1. Sequences flanking the lox site allow transplacement by homologous recombination with the wild-type viral DNA.

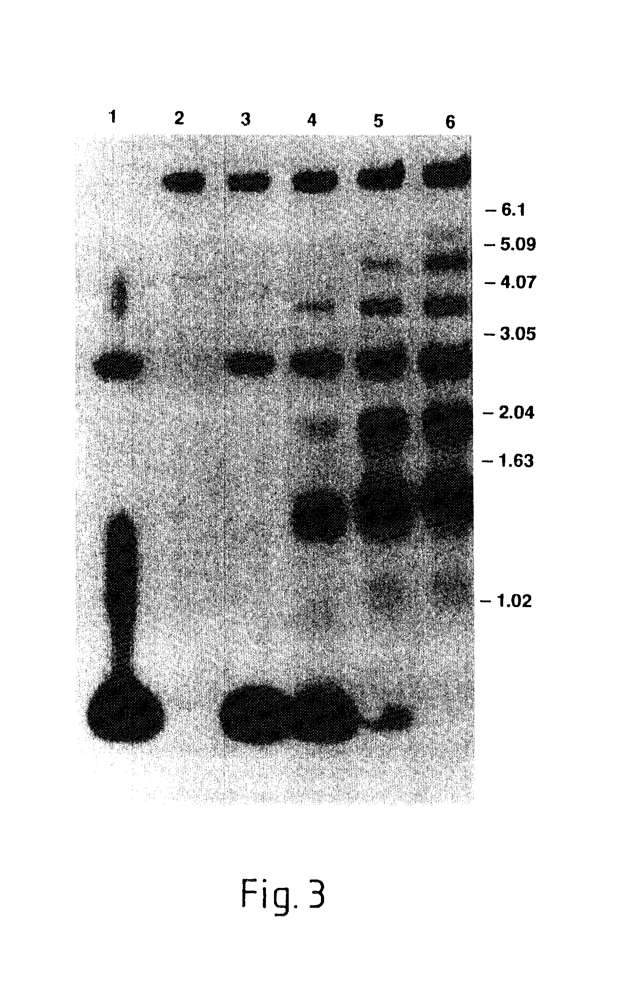

Figure 3 relates to in vitro Cre mediated recombination of pKSlox and p36Clox. Plasmids pKSlox (21g) and p36Clox (21g) were linearised with PvuII and Sal1 respectively and incubated together in the presence or absence of Cre recombinase. The products of the reaction were analysed by agarose gel electrophoresis and Southern blotting using the 445bp PvuII fragment from pKSlox as a probe. Lanel; pKSlox, lane 2; p36Clox, lane3; pKSlox + p36Clox, lanes 4-6; pKSlox + p36Clox in the presence of 1,2, or 4 units of Cre recombinase respectively. The positions of molecular weight markers (in kbp) are shown on the right.

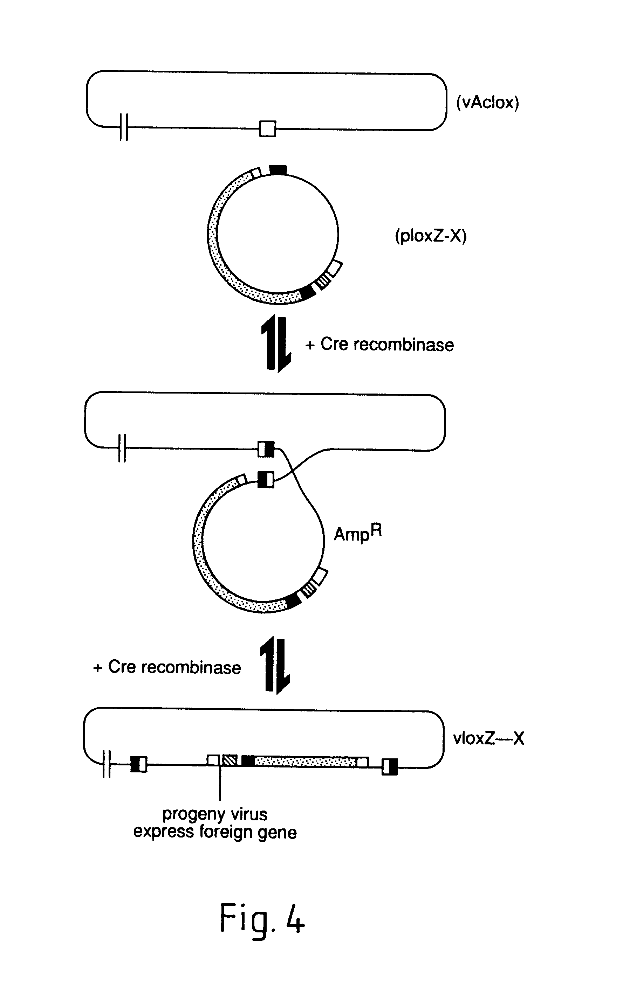

Figure 4 provides a diagrammatic representation of Cre mediated recombination at loxP sites. The viral genome (vAclox) is shown as a broken circle containing a loxP site (open box). The key for ploxZ is as for Figure 2a with 'X' indicating the presence of an heterologous gene. The recombinant virus is shown containing two hybrid loxP sites (shown as black and white boxes). The arrows indicate the reversable nature of the reaction in the presence of Cre recombinase.

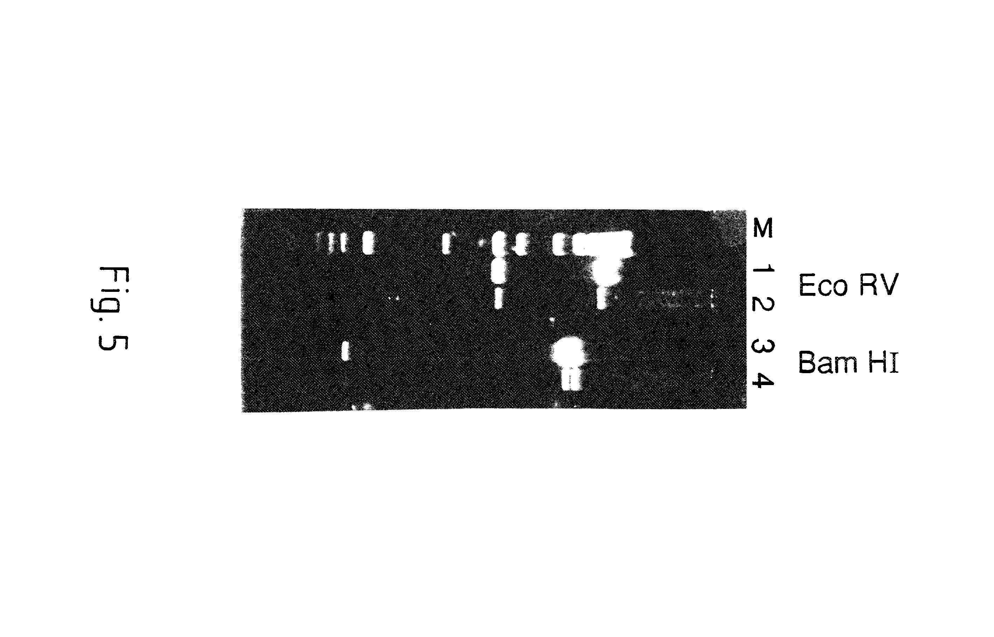

Figure 5 provides a Cre-mediated recovery of ploxZ from vloxZ DNA. DNA prepared from cells infected with vloxZ or control virus was incubated with Cre and the reactions used to transform E.coli. DNA was prepared from ampicillin resistant colonies and analysed by restriction endonuclease digestion followed by agarose gel electrophoresis; Lanes 1 and 3; control ploxZ, lanes 2 and 4; DNA miniprep from E.coli transformed with vloxZ DNA incubated with Cre. M : Ikbp ladder molecular weight marker.

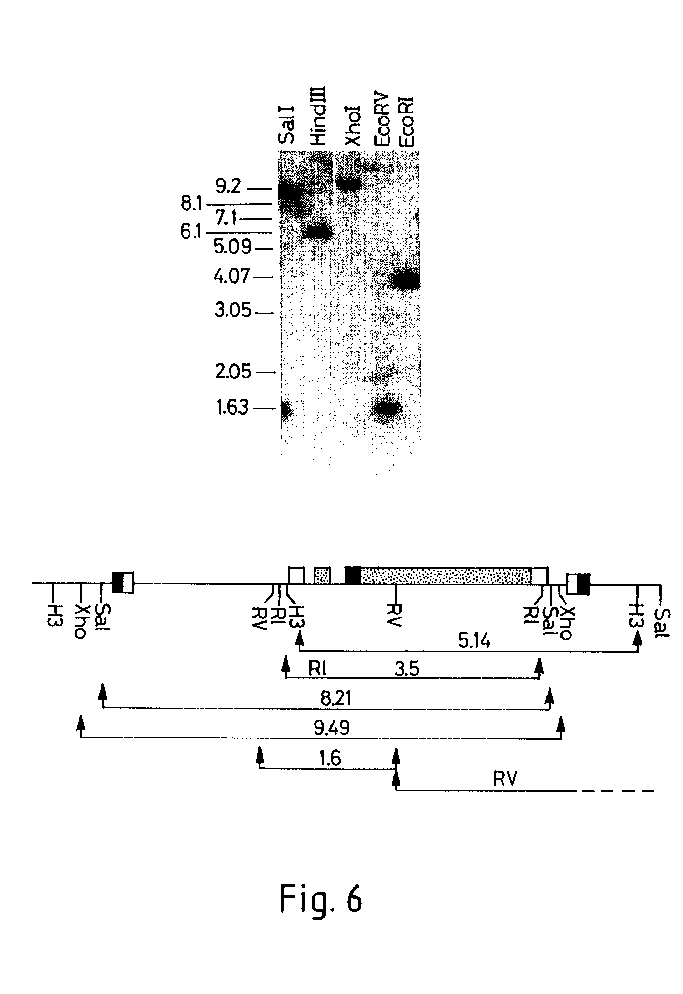

Figure 6 shows a Southern analysis of vloxZ recombinants. DNA from cells infected with vloxZ was digested with a number of restriction endonucleases, transferred to a nylon membrane and probed with a fragment corresponding to the lacZ gene. The top half of the Figure shows an autoradiograph of such a blot together with the positions of molecular weight markers (in kbp) and the restriction enzymes used. Below is a representation of the predicted structure of vloxZ with restriction sites and the sizes (in kbp) of cross-hybridizing fragments. The hybrid lox sites (black and white boxes) flank the plasmid sequences (key as for Figure 2a) H3; HindIII, Xho; XhoI, Sal; SalI, RV; EcoRV, RI; EcoRI.

The following Examples are provided in illustration of the present invention, in which

(i) Spodoptera frugiperda (Sf) cells were maintained in spinner flasks in TC100 medium (Flow Laboratories Ltd., Woodcock Hill, Harefield Road, Rickmansworth, Herts. WD3 1PQ) supplemented with 10% foetal calf serum and 0.1mg/ml gentamicin (Sigma Chemical Company Ltd., Fancy Road, Poole, Dorset BH17 7NH). Wild type and recombinant viruses were grown and plaque purified according to the procedures described previously. (Summers et al., Texas Agricultural Experimental Station Bulletin No. 1555, 1987).

(ii) All plasmid DNA manipulations and Southern blotting techniques were done as described in reference in Maniatis et al., Molecular Cloning: A Laboratory Manual, 1982, Cold Spring Habor Laboratory. DNA restriction and modification enzymes were purchased from GIBCO-BRL, Unit 4, Cowley Mill Trading Estate, Longbridge Way, Uxbridge, Middlesex UB8 2YG and Boehringer Mannheim UK (Diagnostics and Biochemicals) Ltd., Bell Lane, Lewes, East Sussex BN7 1LG and used according to the manufacturers instructions. Calf intestinal alkaline phosphatase was purchased from Boehringer Mannheim. Plasmid DNA for transfections was purified on caesium chloride gradients.

## EXAMPLE 1

### Construction of the recombinant transfer vector ploxZ

An oligonucleotide pair corresponding to the sequence of lox (Figure 1) was synthesised and cloned into the KpnI site of the Bluescript+ vector (Stratagene Ltd., Cambridge Innovation Centre, Cambridge Science Park, Milton Road, Cambridge CB4 4GF). This construction was designated pKSlox. A 4.6kbp SalI-HindIII fragment from pJVPI0Z, a derivative of pJVNheI (Vialard et al., J.Virol., 1990, 64, 37-50), containing the b-galactosidase (lacZ) gene under the transcriptional regulation of the very late viral P10 promoter and the very late viral polyhedrin promoter immediately upstream of a unique NheI cloning site, was cloned between the SalI and HindIII sites of pKSlox. The resulting construct was designated ploxZ (Figure 2a).

## EXAMPLE 2

### Construction of the recombinant virus vAclox

An oligonucleotide pair corresponding to the sequence of lox (Figure 1) was synthesised and cloned between the EcoRV and KpnI sites of the baculovirus transfer vector p36C (Page, Nucleic Acids Res., 1989, 17, 454). The resulting construct was designated p36Clox (Figure 2b). To obtain the recombinant virus (vAclox), Sf cells were irradiated with short wave ultra-violet light as described (Peakman et al., Nucleic Acids Res., 1989, 17, 5403) and transfected with 41g p36Clox and 1lg wild type viral DNA using the Transfectam reagent (10lg/ml - Stratagene Ltd., Cambridge Innovation Centre, Cambridge Science Park, Milton Road, Cambridge CB4 4G) in 5ml serum free medium for 6 hours. The transfection medium was replaced with 5ml complete medium and the incubation continued at 27°C. After seventy two hours, the supernatant was harvested and the progeny virus were screened in a standard agarose overlay assay (Brown, et al., J.Gen.Virol, 1977, 36, 361-364). Plaques were screened visually for the absence of polyhedra. Potential polyhedrin negative recombinant plaques were purified to homogeneity by successive rounds of agarose overlay assay. The presence of the lox insertion was confirmed by purifying DNA from 2 million infected cells followed by Southern analysis. High titre recombinant virus (>$10^9$ plaque forming units/ml) was prepared by infecting monolayer cultures at 27°C.

## EXAMPLE 3

### In vitro recombination reactions and screening of products:- detection of b-galactosidase activity

The plasmid ploxZ and viral vAclox DNA were mixed in a buffer containing 50mM Tris.HCl pH7.5, 33mM NaCl, 10mM $MgCl_2$ and 100lg/ml BSA. Reactions were incubated at 37°C in the presence or absence of Cre recombinase (NEN-DuPont (UK) Ltd., Biotechnology Systems Divisions, Wedgewood Way, Stevenage, Herts. SG1 4QN). The reactions were terminated by incubation at 70°C for 15min. After cooling to room temperature, the reactions were mixed with the Transfectam reagent and used to transfect a monolayer of $2 \times 10^6$ Sf cells

7

as described in Example 2 and progeny virus were screened in an overlay assay. The presence of integrated ploxZ plasmid in the viral progeny was detected by the additon of 40ll of a solution of 40mg/ml (in DMSO) of the chromogenic indicator X-Gal (Boehringer Mannheim UK (Diagnostics and Biochemicals) Ltd., Bell Lane, Lewes, East Sussex BN7 1LG), 72hpi followed by incubation at 27°C until vivid blue plaques were visible (typically 2-4h.). These recombinant viruses were designated vloxZ. Non-recombinant viruses were visualised by the addition of 250ll of neutral red solution (BDH (Head Office); Merck Ltd., Broom Road, Poole, Dorset BH12 4NNN) to the overlay 72hpi followed by incubation at 27°C for 1h. The plaques were destained at 4°C overnight. Recombinant viruses were purified to homogeneity (100% blue staining plaques) by 2-3 successive rounds of agarose overlay assay.

## EXAMPLE 4

Assays of b-galactosidase activity of cells infected with vloxZ

Monolayers of Sf cells (2X10^6) were infected with virus at a multiplicity of infection (MOI) of 1 and incubated for 3 days at 27°C. After infection, the cells were rinsed with ice-cold PBS and scraped from the plates in 0.5ml ice-cold PBS. The cells were lysed by the addition of 30ll of toluene and 30ll of a 1%w/v solution of sodium deoxycholate and the lysates assayed for b-galactosidase activity as previously described (Maniatis et al.; ibid.) using the chromogenic indicator 2-nitrophenyl-b-D galactopyranoside (ONPG -Boehringer Mannheim), The units of activity were expressed as lmoles ONPG hydrolysed per minute per million virally infected cells.

## EXAMPLE 5

Recovery of ploxZ plasmid DNA from vloxZ

Monolayer Sf cells were infected with purified vloxZ virus at a MOI of 1. Total DNA, containing viral and host cell DNA, was prepared from the infected cells 72hpi (Summers et al., Texas Agricultural Experiment Station Bulletin No. 1555). Various amounts of DNA were incubated in Cre buffer at 37°C for 30min. in the presence or absence of 1 unit Cre recombinase. After termination of the reaction, the DNA mixture was used to transform Escherichia coli DH5a and plated on L-agar plates containing 100lg/ml ampicillin (Sigma Chemical Co. Ltd). DNA was prepared from resulting bacterial colonies and analysed by agarose gel electrophoresis.

## EXAMPLE 6

Assay of in vitro Cre mediated recombination between pKSlox and p36Clox

In order to test the functionality of the lox sequences in the vectors pKSlox and p36Clox in vitro Cre mediated recombination assays were initially attempted. Plasmids pKSlox and p36Clox were digested with SalI and PvuII respectively. The linear DNAs were mixed in the absence or presence of varying amounts of Cre recombinase and incubated for 30min. at 37°C. After termination of the reaction, the products were electrophoresed on an agarose gel and analysed by Southern blotting (Figure 3) using a 445bp PvuII probe corresponding to the lox containing fragment of pKSlox. In the presence of Cre recombinase, recombination events lead to the appearance of hybridizing bands larger than the substrate 445bp species. In the reactions containing higher levels of Cre, the bands corresponding to recombinant products are more intense and, in addition, a number of higher molecular weight species are visible (lanes 5 and 6). It is difficult to assign structures to these recombinant species since each new recombinant becomes a fresh substrate for Cre recombinase. However, it is clear that the recombination is dependent on both the presence and amount of Cre recombinase and also that the lox sequences in this context are an efficient substrate for the Cre protein.

## EXAMPLE 7

Stoichiometry and kinetics of Cre mediated recombination

Because the Cre mediated recombination is an enzymic reaction (Figure 4) we investigated the relationship between efficiency of recombination and both the ratio of the viral and plasmid DNAs and the time of incubation with the Cre recombinase prior to transfection.

In the first experiment (Table 1), the amount of substrate was kept constant and the time of incubation with Cre varied. Initially, 5lg of vAclox and 1lg ploxZ were mixed in a final volume of 60ll in the presence of 2 units of Cre recombinase. At each time point, 10ll aliquots were removed and the reaction terminated. The DNA was used to transfect Sf monolayers as described and the viral progeny screened for b-galactosidase activity. The results, expressed as recombinants/lg ploxZ plasmid DNA, indicate that the reaction is virtually at equilibrium after 10 minutes with only a small increase in the number of recombinants at later time points. It is possible that after very long incubation times the number of recombinants would decrease, since each new recombinant species is itself a fresh substrate for Cre (see Figure 4) and the resulting recombinants may not be viable.

In the second experiment, (Table 2), the ratio of vAclox to ploxZ was varied. Viral DNA (vAvlox - 0.5ll) was mixed with ten-fold dilutions of ploxZ in a final volume of 10ll and the DNA incubated for 20 minutes at 37°C in the presence of Cre recombinase. After termination, the DNA was used to transfect Sf monolayers and the viral progeny screened for recombinants expressing b-galactosidase. The results, adjusted according to the amount of plasmid DNA used, indicate increased viable recombinants with lower amounts of ploxZ but a lower percentage of recombinant virus to non-recombinant virus. This may be due to unfavourable recombination events in the reactions containing higher concentrations of ploxZ (eg. ploxZ-ploxZ) or secondary and tertiary recombination with products of previous integrations which are non-viable. The observation that the total number of viral progeny was considerably reduced in the reactions containing the higher concentrations of ploxZ supports this possibility. These results suggest that very small amounts of transfer vector DNA are sufficient to generate significant numbers of recombinant viruses. Representative blue plaques were purified to homogeneity and remain stable through successive rounds of agarose overlay assay.

Although it is clear from the coloured plaques that the infected cells are expressing b-galactosidase, Sf monolayers were infected with progeny virus to compare the levels of expression between the various constructs and to ensure that Sf cells do not possess an endogenous enzyme that could produce spurious results. Not surprisingly, cells infected with viral progeny generated from a clear plaque do not express b-galactosidase at detectable levels. Uninfected Sf cells similarly do not possess a detectable endogenous b-galactosidase activity. In contrast, cells infected with vloxZ express greater than 50,000 units of b-galactosidase. This very high level of expression explains why blue plaques are visible so soon after the addition of X-gal. This is an advantageous feature since recombinant plaques can be confidently identified immediately following the plaque assay rather than the following day after staining and destaining with neutral red.

### Table 1

| Time (min) | Number of recombinants (X10$^6$)/lg plasmid DNA |
| --- | --- |
| 0 | 0 |
| 10 | 5.6 |
| 20 | 6.8 |
| 40 | 6.6 |
| 80 | 7.8 |

Assay of number of recombinant virus with time of incubation with Cre recombinase. Plasmid ploxZ and viral vAclox were incubated with Cre recombinase and then used to transfect Sf cell monolayers. The progeny virus were screened in a standard agarose overlay assay. The chromogenic indicator X-gal was added 72hpi and recombinants identified as blue plaques.

## Table 2.

| Viral DNA (lg) | ploxZ (lg) | No. recombinants $(X10^6)$/lg plasmid DNA | % viral progeny expressing b-gal. |
|---|---|---|---|
| | 0 | 0 | 0 |
| | 3 | 2.1 | 23 |
| vAclox (0.5) | 0.3 | 27 | 49 |
| | 0.03 | 27 | 2 |
| | 0.003 | 54 | 0.6 |
| wild type (0.5) | 0 | 0 | 0 |
| | 3 | 0 | 0 |

Titration of vAclox with ploxZ. 0.5lg vAclox was incubated with 10-fold dilutions of ploxZ for 20 minutes at 37°C. After standard transfection and agarose overlay assay, the chromogenic indicator, X-gal, was added and recombinants identified as blue plaques. Wild type Autographa californica viral DNA was included as a negative control.

EXAMPLE 8

Recovery of ploxZ DNA from cells infected with vloxZ

If integration of ploxZ is genuinely site specific and mediated by Cre recombinase, the resulting progeny virus, vloxZ, should contain the sequences of ploxZ flanked by two lox sites (Figure 4). The reversibility of the Cre mediated recombination was tested by incubating vloxZ DNA with Cre recombinase to recover the plasmid DNA. Sf monolayers were infected with vloxZ or a control virus. Total DNA (only a fraction of which is viral) from infected cells was incubated with Cre and the incubation mix used to transform Escherichia coli DH5a cells (Table 3). The recovery of ampicillin resistant clones from vloxZ, which is absolutely dependent on the presence of Cre, suggests the integration has occurred as predicted and that both lox sites are intact. As a final confirmation of the integrity of the recovered plasmid, DNA was prepared from the ampicillin resistant bacterial colonies and analysed by gel electrophoresis after digestion with diagnostic restriction enzymes (Figure 5). Digestion of the recovered DNA with BamHI and EcoRV shows it to be identical to the starting plasmid ploxZ. This is further confirmation that the integration has occurred as predicted and that inserts can be conveniently and rapidly recovered from viral DNA.

**Table 3.**

| Viral DNA | Cre | No. Amp$^r$ clones. |
|---|---|---|
| Wild type (0.05lg) | - | 0 |
| Wild type (0.05lg) | + | 0 |
| vloxZ (0.05lg) | - | 0 |
| vloxZ (0.05lg) | + | 7 |
| (0.1lg) | + | 5 |
| 0.5lg) | + | 14 |
| (1.0lg) | + | 17 |
| (5.0lg) | + | 24 |

Recovery of ploxZ DNA from vloxZ. Total DNA from infected Sf cells was incubated with Cre for 30 minutes at 37°C and then used directly to transform Escherichia coli DH5a and plated onto L-agar plates containing 100lg/ml ampicillin. The plates were incubated overnight at 37°C and the colonies counted.

EXAMPLE 9

Southern analysis of vloxZ recombinants

As further confirmation of the structure of the recombinant virus, vloxZ DNA from purified high titre viral progeny was analysed by Southern blotting (Figure 6). DNA from 2 million cells infected with vloxZ was prepared and digested with a number of diagnostic restriction enzymes and Southern blots of these digests probed with a labelled fragment corresponding to the lacZ gene present in ploxZ. The sizes of the bands on the blot are consistent with the structure predicted in Figure 6. Thus, integration of the transfer vector, ploxZ, in the presence of Cre recombinase is site specific and not simply a random integration anywhere in the viral chromosome.

Suppliers.

i) **DuPont (UK) Ltd.** Biotechnology Systems Divisions, Wedgewood Way, Stevenage, Herts. SG1 4QN
ii) **Stratagene Ltd.** Cambridge Innovation Centre, Cambridge Science Park, Milton Rd., Cambridge. CB4 4GF
iii) **Flow Laboratories Ltd.** Woodcock Hill, Harefield Rd., Rickmansworth, Herts. WD3 1PQ
iv) **Sigma Chemical Company Ltd.** Fancy Rd., Poole, Dorset. BH17 7NH
v) **Gibco Ltd.** Unit4, Cowley Mill Trading Estate, Longbridge Way, Uxbridge, Middlesex. UB8 2YG
vi) **Boehringer Mannheim UK** (Diagnostics and Biochemicals) Ltd., Bell Lane, Lewes, East Sussex. BN7 1LG
vii) **BDH.** (Head Office); Merck Ltd, Broom Rd., Poole Dorset. BH12 4NN

**Claims**

1. A baculovirus transfer vector, which contains a restriction site for insertion of a DNA sequence encoding an heterologous polypeptide, regulatory DNA elements to provide for expression of the DNA sequence, when inserted into the restriction site, and a loxP DNA sequence to act as a substrate for the Cre recombinase protein.

2. A baculovirus transfer vector as claimed in claim 1 wherein the loxP DNA sequence is substantially the same as that which follows:

5′ CCTTAATATAACTTCGTATAATGTATGCTATACGAAGTTATTAGGTCG 3′

3′ GGAATTATATTGAAGCATATTACATACGATATGCTTCAATAATCCAGC 5′

3. A recombinant DNA molecule comprising a baculovirus transfer vector as claimed in claim 1 and a DNA sequence encoding an heterologous polypeptide inserted into the restriction site of the vector.

4. A baculovirus which contains a loxP DNA sequence to act as a substrate for the Cre recombinase protein.

5. A recombinant baculovirus produced by homologous recombination of baculovirus, which contains a loxP DNA sequence to act as a substrate for the Cre recombinase protein and a recombinant DNA molecule as claimed in claim 3.

6. An insect cell transfected with a recombinant baculovirus as claimed in claim 5.

7. An insect cell as claimed in claim 6 wherein the insect cell is Spodoptera frugiperda.

8. A process for the production of an heterologous polypeptide which comprises culturing an insect cell with a recombinant baculovirus as claimed in claim 5.

9. A vaccine for conferring immunity comprising a polypeptide produced according to the process as claimed in claim 8.

10. A vaccine as claimed in claim 9 in association with a physiologically acceptable carrier or diluent possibly in admixture with other agents.

11. A test kit for detecting antibody reactive with polypeptide comprising:
    (1) polypeptide produced by the process as claimed in claim 8 labelled with enzyme;
    (2) a substrate for the enzyme;
    (3) means providing a surface on which polypeptide is immobilised; and
    (4) optionally, washing solutions and/or buffers.

12. A test kit for detecting polypeptide comprising:
    (1) antibody reactive with polypeptide produced by the process as claimed in claim 8 labelled with enzyme;
    (2) a substrate for the enzyme;
    (3) means providing a surface on which antibody is immobilised; and
    (4) optionally, washing solutions and/or buffers.

EP 0 542 466 A2

Blunt - EcoRV                                                                KpnI

5′ CCTTAATATAACTTCGTATAATGTATGCTATACGAAGTTATTAGGTCGGTAC   3′
   GGAATTATATTGAAGCATATTACATACGATATGCTTCAATAATCCAGC

vAc36Clox.


KpnI                                                                         KpnI

5′      CCCTTAATATAACTTCGTATAATGTATGCTATACGAAGTTATTAGGTCGGTAC 3′
   CATGGGGAATTATATTGAAGCATATTACATACGATATGCTTCAATAATCCAGC

ploxZ.


Fig.1

Fig.2a

Fig.2b

Fig. 3

(vAclox)

(ploxZ-X)

+ Cre recombinase

Amp$^R$

+ Cre recombinase

vloxZ—X

progeny virus
express foreign gene

Fig. 4

Fig. 5

Fig. 6